(19) Europäisches Patentamt
European Patent Office
Office européen des brevets

(11) Veröffentlichungsnummer: **0 301 348 B1**

(12) **EUROPÄISCHE PATENTSCHRIFT**

(45) Veröffentlichungstag der Patentschrift: **03.02.93**

(51) Int. Cl.5: **C07D 213/80**, C07D 213/82, C07D 213/85, A23K 1/16

(21) Anmeldenummer: **88111504.2**

(22) Anmeldetag: **18.07.88**

Die Akte enthält technische Angaben, die nach dem Eingang der Anmeldung eingereicht wurden und die nicht in dieser Patentschrift enthalten sind.

(54) **Substituierte Pyridylethanolamine, Verfahren zu ihrer Herstellung und ihre Verwendung als Leistungsförderer bei Tieren.**

(30) Priorität: **29.07.87 DE 3725084**

(43) Veröffentlichungstag der Anmeldung:
**01.02.89 Patentblatt 89/05**

(45) Bekanntmachung des Hinweises auf die Patenterteilung:
**03.02.93 Patentblatt 93/05**

(84) Benannte Vertragsstaaten:
**AT BE CH DE ES FR GB GR IT LI NL SE**

(56) Entgegenhaltungen:
**EP-A- 0 049 728**
**EP-A- 0 170 538**
**EP-A- 0 209 025**
**EP-A- 0 225 600**
**EP-A- 0 244 728**

(73) Patentinhaber: **BAYER AG**
**W-5090 Leverkusen 1 Bayerwerk(DE)**

(72) Erfinder: **Lindel, Hans, Dr.**
**Carl-Duisberg-Strasse 321**
**W-5090 Leverkusen 1(DE)**
Erfinder: **Hallenbach, Werner, Dr.**
**Kleiststrasse 10**
**W-4018 Langenfeld(DE)**
Erfinder: **Berschauer, Friedrich, Prof. Dr.**
**Claudiusweg 9**
**W-5600 Wuppertal 1(DE)**

**EP 0 301 348 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft substituierte Pyridylethanolamine, Verfahren zu ihrer Herstellung, Zwischenprodukte zur Durchführung dieser Verfahren, sowie ihre Verwendung als Leistungsförderer bei Tieren.

Die Verwendung von Futtermittelzusätzen zur Erzielung höherer Gewichtszuwächse und verbesserter Futterausnutzung wird in der Tierernährung insbesondere bei der Mast von Schweinen, Rindern und Geflügel praktiziert.

Heteroarylethylamine sind bereits bekannt geworden. Die Verbindungen besitzen beta-sympathomimetische Wirkungen (DE-OS 1 811 833, 2 603 600, EP-OS 120 770, US-PS 4 358 455). Es ist jedoch nichts über ihre Eignung als Leistungsförderer bei Tieren bekannt. Aus EP-OS 170 538 ist die Verbindung der Formel

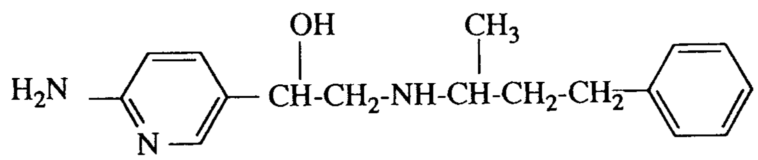

sowie ihre Verwendung als Wachstumsförderer für Tiere bekannt. Ihre Wirkung befriedigt jedoch nicht.

Es wurde gefunden:

1. Neue substituierte Pyridylethanolamine der Formel I,

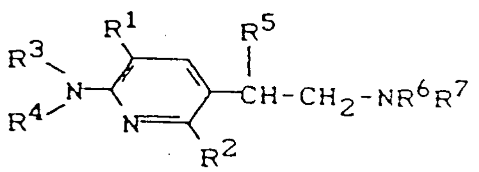

I

in welcher

| | |
|---|---|
| $R^1$ | für CN, $-COOR^8$, $-CONR^9R^{10}$ steht, |
| $R^2$ | für $C_{1-4}$-Alkyl steht, |
| $R^3$ | für Wasserstoff oder $C_{1-4}$-Alkyl steht, |
| $R^4$ | für Wasserstoff oder $C_{1-4}$-Alkyl steht oder $R^3$ und $R^4$ gemeinsam mit dem Stickstoffatom, an das sie gebunden sind, für einen gegebenenfalls durch Halogen oder $C_{1-4}$-Alkyl substituierten Heterocyclus aus der Reihe Pyrrolidin, Pyrrol, Piperidin, Morpholin stehen, |
| $R^5$ | für OH, $C_{1-6}$-Alkoxy oder Acyloxy steht und Acyloxy für Oxycarbonyl-$C_{1-6}$-alkyl, Oxycarbonylphenyl, Oxysulfonyl-$C_{1-6}$-alkyl, Olxysulfonylphenyl steht. |
| $R^6$ | für Wasserstoff oder $C_{1-6}$-Alkyl steht, |
| $R^7$ | für Wasserstoff, gegebenenfalls durch 1 bis 5 Halogenatome substituiertes $C_{1-6}$-Alkyl Cycloalkyl mit bis zu 6 C-Atomen $C_{1-6}$-Alkylphenyl, das gegebenenfalls durch Halogen, $C_{1-4}$-Alkyl, Hydroxy, $C_{1-4}$-Alkoxy, gegebenenfalls halogensubstituiertes Methylendioxy oder Ethylendioxy substituiert sein kann; $C_{1-6}$-Alkylphenoxy, das gegebenenfalls durch Halogen, $C_{1-4}$-Alkyl, Hydroxy, $C_{1-4}$-Alkoxy, gegebenenfalls halogensubstituiertes Methylendioxy oder Ethylendioxy substituiert sein kann, steht, |
| $R^6$ und $R^7$ | können gemeinsam mit dem Stickstoffatom, an das sie gebunden sind, für einen 5- bis 6-gliedrigen gesättigten oder ungesättigten heterocyclischen Rest stehen, der gegebenenfalls noch weitere Heteroatome aus der Reihe N, O, S enthalten kann, |
| $R^8$ | für $C_{1-6}$-Alkyl, |
| $R^9$ und $R^{10}$ | unabhängig voneinander für Wasserstoff oder $C_{1-4}$-Alkyl stehen, |

sowie ihre physiologisch verträglichen Salze und ihre N-Oxide.

2. Verfahren zur Herstellung der substituierten Pyridylethanolamine der Formel I

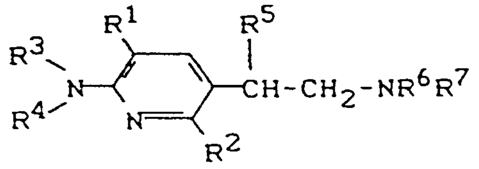

I

in welcher

$R^1$ für CN, $-COOR^8$, $-CONR^9R^{10}$ steht,

$R^2$ für $C_{1-4}$-Alkyl steht,

$R^3$ für Wasserstoff oder $C_{1-4}$-Alkyl steht,

$R^4$ für Wasserstoff oder $C_{1-4}$-Alkyl steht oder $R^3$ und $R^4$ gemeinsam mit dem Stickstoffatom, an das sie gebunden sind, für einen gegebenenfalls durch Halogen oder $C_{1-4}$-Alkyl substituierten Heterocyclus aus der Reihe Pyrrolidin, Pyrrol, Piperidin, Morpholin stehen,

$R^5$ für OH, $C_{1-6}$-Alkoxy oder Acyloxy steht und Acyloxy für Oxycarbonyl-$C_{1-6}$-alkyl, Oxycarbonylphenyl, Oxysulfonyl-$C_{1-6}$-alkyl, Oxysulfonyl-$C_{1-6}$-alkyl, Oxysulfonylphenyl steht,

$R^6$ für Wasserstoff oder $C_{1-6}$-Alkyl steht,

$R^7$ für Wasserstoff, gegebenenfalls durch 1 bis 5 Halogenatome substituiertes $C_{1-6}$-Alkyl Cycloalkyl mit bis zu 6 C-Atomen $C_{1-6}$-Alkylphenyl, das gegebenenfalls durch Halogen, $C_{1-4}$-Alkyl, Hydroxy, $C_{1-4}$-Alkoxy, gegebenenfalls halogensubstituiertes Methylendioxy oder Ethylendioxy substituiert sein kann; $C_{1-6}$-Alkylphenoxy, das gegebenenfalls durch Halogen, $C_{1-4}$-Alkyl, Hydroxy, $C_{1-4}$-Alkoxy, gegebenenfalls halogensubstituiertes Methylendioxy oder Ethylendioxy substituiert sein kann, steht,

$R^6$ und $R^7$ können gemeinsam mit dem Stickstoffatom, an das sie gebunden sind, für einen 5- bis 6-gliedrigen gesättigten oder ungesättigten heterocyclischen Rest stehen, der gegebenenfalls noch weitere Heteroatome aus der Reihe N, O, S enthalten kann,

$R^8$ für $C_{1-6}$-Alkyl,

$R^9$ und $R^{10}$ unabhängig voneinander für Wasserstoff oder $C_{1-4}$-Alkyl stehen,

sowie ihre physiologisch verträglichen Salze und ihre N-Oxide,

dadurch gekennzeichnet, daß man substituierte Pyridylhalogenmethylketone der Formel II,

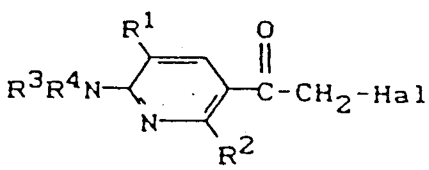

II

in welcher

$R^1$, $R^2$, $R^3$, $R^4$ die oben angegebenen Bedeutungen haben,

Hal für Halogen steht,

mit Aminen der Formel III,

$HNR^6R^7$ III

in welcher

$R^6$ und $R^7$ die oben angegebene Bedeutung haben,

umsetzt und anschließend die Carbonylgruppe reduziert und danach gegebenenfalls acyliert oder alkyliert.

3. Substituierte Pyridylhalogenmethylketone der Formel II,

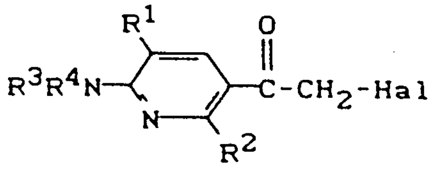

II

in welcher
$R^1$, $R^2$, $R^3$, $R^4$, Hal die weiter oben angegebene Bedeutung haben,
sowie ihre physiologisch verträglichen Salze und ihre N-Oxide.

4. Verfahren zur Herstellung der Pyridylhalogenmethylketone der Formel II,

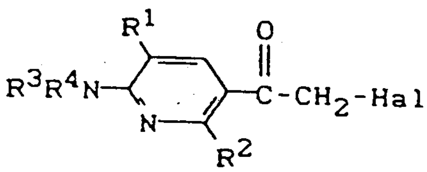

II

in welcher
$R^1$, $R^2$, $R^3$, $R^4$, Hal die weiter oben angegebene Bedeutung haben,
dadurch gekennzeichnet, daß man substituierte Pyridylmethylketone der Formel IV,

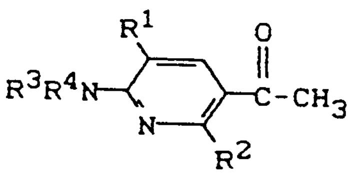

IV

in welcher
$R^1$, $R^2$, $R^3$, $R^4$ die oben angegebene Bedeutung haben,
halogeniert.

5. Substituierte Pyridylmethylketone der Formel IV,

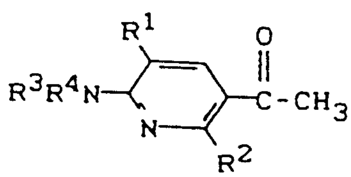

IV

in welcher
$R^1$, $R^2$, $R^3$, $R^4$ die weiter oben angegebene Bedeutung haben,
sowie ihre physiologisch verträglichen Salze und ihre N-Oxide.

6. Verfahren zur Herstellung der substituierten Pyridylmethylketone der Formel IV,

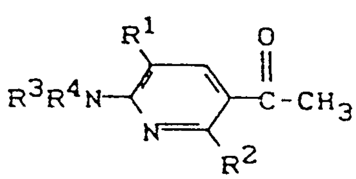

IV

in welcher
$R^1$, $R^2$, $R^3$, $R^4$ die weiter oben angegebene Bedeutung haben,
dadurch gekennzeichnet, daß man Pyridylmethylketone der Formel V,

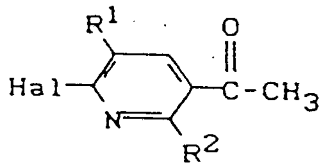

V

in welcher
$R^1$ und $R^2$ die oben angegebene Bedeutung haben,
Hal für Halogen steht,
mit Aminen der Formel VI,

$H\text{-}NR^3R^4$ VI

in welcher
$R^3$ und $R^4$ die oben angegebene Bedeutung haben,
umsetzt.

7. Pyridylmethylketone der Formel V,

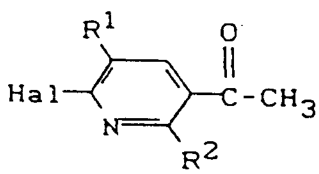

V

in welcher
$R^1$, $R^2$ und Hal die weiter oben angegebene Bedeutung haben,
sowie ihre physiologisch verträglichen Salze und ihre N-Oxide.

8. Verfahren zur Herstellung der Pyridylmethylketone der Formel V,

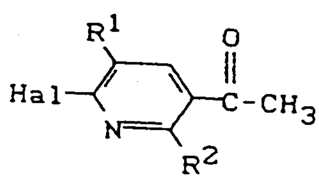

V

in welcher
$R^1$, $R^2$, Hal die oben angegebene Bedeutung haben,
dadurch gekennzeichnet, daß man Verbindungen der Formel VII,

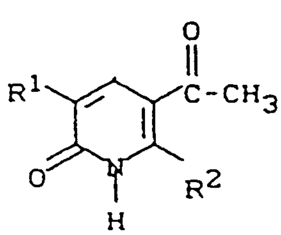

VII

in welcher
$R^1$ und $R^2$ die oben angegebene Bedeutung haben,
mit Phosphoroxychlorid, gegebenenfalls in Gegenwart von Säurebindemitteln, umsetzt.

Die Verbindungen der Formel I Können in Form ihrer Tautomeren vorliegen. Beispiele dafür sind:

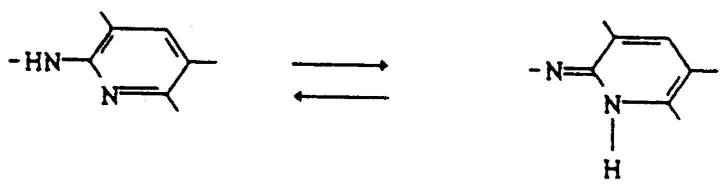

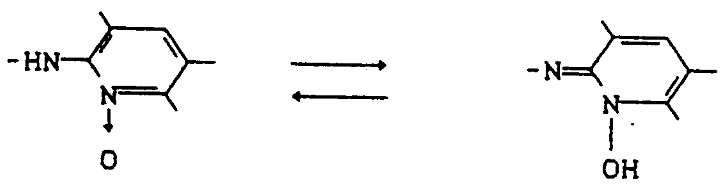

Die Verbindungen der Formel I können auch in Form ihrer Racemate oder enantiomeren Formen vorliegen.

Physiologisch verträgliche Salze der Verbindungen der Formel I können mit folgenden Säuren gebildet werden:
Salzsäure, Schwefelsäure, Phosphorsäure, Perchlorsäure, Brom-, Iodwasserstoffsäure, Salpetersäure, Essigsäure, Oxalsäure, Malonsäure, Bernsteinsäure, Ascorbinsäure, Äpfelsäure, Weinsäure, Maleinsäure, Fumarsäure, Methansulfonsäure, Benzoesäure, substituierte Benzoesäuren, Ameisensäure, Toluolsulfonsäure, Benzolsulfonsäure, Phthalsäure, Naphthalinsulfonsäure, Nikotinsäure, Palmitinsäure, Embonsäure.

Bevorzugt sind Verbindungen der Formel I
in welcher

- $R^1$ für CN steht,
- $R^2$ für $C_{1-4}$-Alkyl steht,
- $R^3$ für Wasserstoff oder $C_{1-4}$-Alkyl steht,
- $R^4$ für Wasserstoff oder $C_{1-4}$-Alkyl steht oder $R^3$ und $R^4$ gemeinsam mit dem Stickstoffatom, an das sie gebunden sind, für einen gegebenenfalls substituierten Heterocyclus aus der Reihe Pyrrolidin, Pyrrol, Piperidin, Morpholin stehen,
- $R^5$ für OH, $C_{1-6}$-Alkoxy oder Acyloxy steht und Acyloxy für Oxycarbonyl-$C_{1-6}$-alkyl, gegebenenfalls substituiertes Oxycarbonylphenyl, Oxysulfonyl-$C_{1-6}$-alkyl, gegebenenfalls substituiertes Oxysulfonylphenyl steht,
- $R^6$ für Wasserstoff oder $C_{1-6}$-Alkyl steht,
- $R^7$ für Wasserstoff, geaebenenfalls durch 1 bis 5 Halogenatome substituiertes $C_{1-6}$-Alkyl, $C_{1-6}$-Alkylphenyl, das gegebenenfalls durch Halogen, $C_{1-4}$-Alkyl, Hydroxy, $C_{1-4}$-Alkoxy, gegebenenfalls halogensubstituiertes Methylendioxy oder Ethylendioxy substituiert sein kann; $C_{1-6}$-Alkylphenoxy, das gegebenenfalls durch Halogen, $C_{1-4}$-Alkyl, Hydroxy, $C_{1-4}$-Alkoxy, gegebenenfalls halogensubstituiertes Methylendioxy oder Ethylendioxy substituiert sein kann, steht,
- $R^6$ und $R^7$ können gemeinsam mit dem Stickstoffatom, an das sie gebunden sind, für einen gegebenenfalls substituierten 5- bis 6-gliedrigen gesättigten oder ungesättigten heterocyclischen Rest stehen, der gegebenenfalls noch weitere Heteroatome aus der Reihe N, O, S enthalten kann,
- $R^8$ für $C_{1-6}$-Alkyl,
- $R^9$ und $R^{10}$ unabhängig voneinander für Wasserstoff oder $C_{1-4}$-Alkyl stehen.

Besonders bevorzugt sind Verbindungen der Formel I,
in welcher

- $R^1$ für CN steht,
- $R^2$ für Methyl oder Ethyl steht,
- $R^3$ für Wasserstoff, Methyl oder Ethyl steht,
- $R^4$ für Wasserstoff, Methyl oder Ethyl steht oder $R^3$ und $R^4$ gemeinsam mit dem Stickstoffatom, an

das sie gebunden sind, für einen Heterocyclus aus der Reihe Pyrrolidin, Pyrrol, Piperidin, Morpholin stehen, der gegebenenfalls durch Halogen oder $C_{1-4}$-Alkyl substituiert sein kann,

$R^5$ für OH, $C_{1-6}$-Alkoxy, insbesondere Methoxy oder Ethoxy steht,

$R^7$ für Wasserstoff, gegebenenfalls substituiertes $C_{1-6}$-Alkyl, $C_{3-6}$-Cycloalkyl, $C_{1-6}$-Alkylphenyl, Phenyl steht,

$R^6$ für Wasserstoff oder $C_{1-4}$-Alkyl, insbesondere Methyl oder Ethyl steht,

$R^7$ für Wasserstoff, gegebenenfalls durch 1 bis 5 Halogenatome substituiertes $C_{1-6}$-Alkyl, insbesondere Methyl, Ethyl, Propyl, Butyl, Pentyl, Hexyl, wobei insbesondere sekundäre und tertiäre Alkylreste genannt seien, sowie $C_{1-6}$-Alkylphenyl, das gegebenenfalls durch Halogen, insbesondere Fluor oder Chlor, $C_{1-4}$-Alkyl, Hydroxy, $C_{1-4}$-Alkoxy, gegebenenfalls halogensubstituiertes Methylendioxy oder Ethylendioxy substituiert sein kann; $C_{1-6}$-Alkylphenoxy, das gegebenenfalls durch Halogen, insbesondere Fluor oder Chlor, $C_{1-4}$-Alkyl, Hydroxy, $C_{1-4}$-Alkoxy,gegebenenfalls halogensubstituiertes Methylendioxy oder Ethylendioxy substituiert sein kann, steht.

Im einzelnen seien folgende Verbindungen der Formel I genannt:

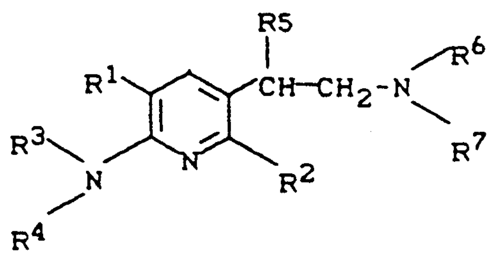

| $R^1$ | $R^2$ | $R^3$ | $R^4$ | $R^5$ | $R^6$ | $R^7$ |
|---|---|---|---|---|---|---|
| C≡N | $CH_3$ | H | H | OH | H | 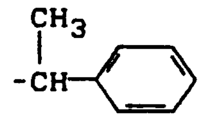 |
| C≡N | $CH_3$ | H | H | OH | $-CH_2-CH_2-O-CH_2-CH_2-$ | |
| C≡N | $CH_3$ | H | H | OH | H | 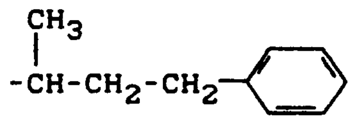 |
| C≡N | $CH_3$ | H | H | OH | H | 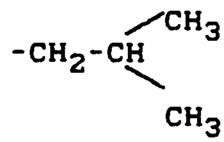 |
| C≡N | $CH_3$ | H | H | OH | H | 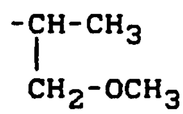 |
| C≡N | $CH_3$ | H | H | OH | H | 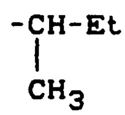 |
| C≡N | $CH_3$ | H | H | OH | $-(CH_2)_5-$ | |
| C≡N | $CH_3$ | H | H | OH | H | 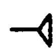 |
| C≡N | $CH_3$ | H | H | OH | H | 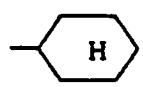 |
| C≡N | $CH_3$ | H | H | OH | H | 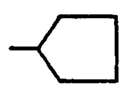 |

| $R^1$ | $R^2$ | $R^3$ | $R^4$ | $R^5$ | $R^6$ | $R^7$ |
|---|---|---|---|---|---|---|
| C≡N | $CH_3$ | $CH_3$ | H | OH | H | 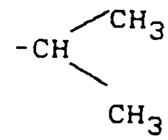 |
| C≡N | $CH_3$ | $CH_3$ | $CH_3$ | OH | H | $-CH(CH_3)_2$ |
| C≡N | $CH_3$ | $-(CH_2)_4-$ | | OH | H | $-CH(CH_3)_2$ |
| C≡N | -Et | H | H | OH | H | $-CH(CH_3)_2$ |
| 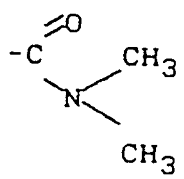 | $CH_3$ | $CH_3$ | $CH_3$ | OH | H | $-CH(CH_3)_2$ |
| 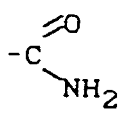 | $CH_2$ | H | H | OH | H | $-CH(CH_3)_2$ |

Bevorzugt seien die Salze mit Salzsäure, Schwefelsäure Phosphorsäure, Oxalsäure, Maleinsäure, Fumarsäure, Malonsäure genannt.

Die neuen Verbindungen der Formel I lassen sich nach dem oben angegebenen Verfahren 2 herstellen.

Setzt man bei Verfahren 2 als Halogenmethylketon der Formel II 2-Methyl-3-chloracetyl-5-cyano-6-amino-pyridin und als Amin der Formel III t-Butylamin ein, läßt sich Verfahren 2 durch folgendes Reaktionsschema wiedergeben:

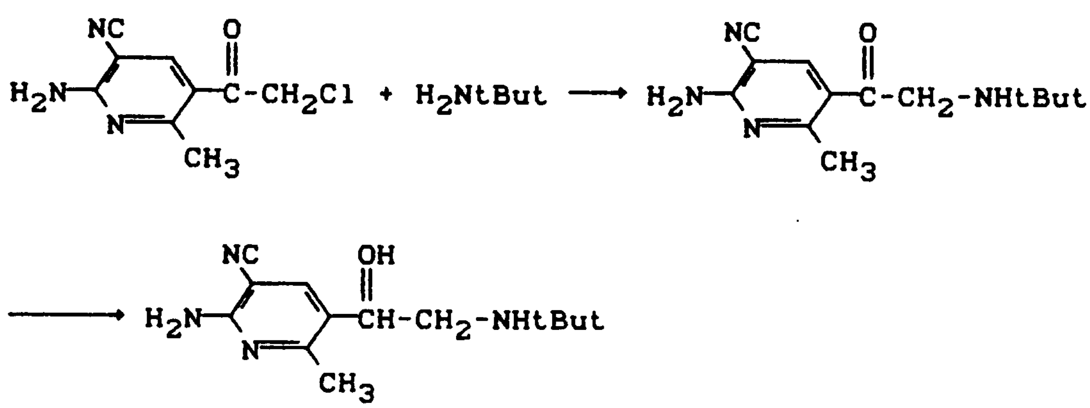

Die Verbindungen der Formel II sind neu. Ihre Herstellung erfolgt nach den weiter unten beschriebenen Verfahren. Bevorzugt sind Verbindungen der Formel II, in der die Substituenten $R^1$, $R^2$, $R^3$, $R^4$ die bei denVerbindungen der Formel I angegebenen bevorzugten Bedeutungen besitzen und Hal für Chlor oder Brom steht.

Im einzelnen seien folgende Pyridylhalogenmethylketone der Formel II genannt:

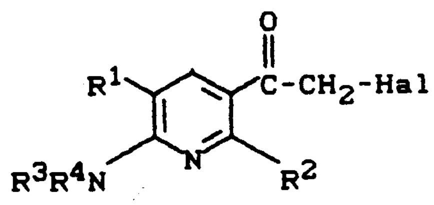

| $R^1$ | $R^2$ | $R^3$ | $R^4$ | Hal |
|---|---|---|---|---|
| $C\equiv N$ | $CH_3$ | H | H | Br |
| $C\equiv N$ | $CH_3$ | $CH_3$ | H | Br |
| $C\equiv N$ | $CH_3$ | $CH_3$ | $CH_3$ | Br |
| $C\equiv N$ | $CH_3$ | $-(CH_2)_4-$ | | Br |
| 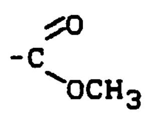 | $CH_3$ | H | H | Br |
| 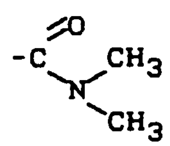 | $CH_3$ | $CH_3$ | $CH_3$ | Br |
| 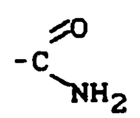 | $CH_3$ | H | H | Br |

Die Amine der Formel III sind bekannt oder lassen sich analog zu bekannten Verfahren herstellen. Die Substituenten $R^6$ und $R^7$ besitzen bevorzugt die weiter oben bei den Verbindungen der Formel I angegebenen bevorzugten Bedeutungen. Im einzelnen seien folgende Verbindungen der Formel III genannt: Ammoniak, Melthylamin, Dimethylamin, Ethylamin, Diethylamin, Methylethylamin, n-Propylamin, i-Propylamin, n-Butylamin, i-Butylamin, tert.-Butylamin, Cyclopentylamin, Cyclohexylamin, Benzylamin, Anilin, Piperidin, Pyrrolidin, Morpholin, 2-Aminopyridin.

Als Reduktionsmittel zur Durchführung des Verfahrens seien folgende Reduktionsmittel genannt:
$H_2$/Katalysator, als Katalysator seien beispielsweise genannt: $PtO_2$, Pd-Aktivkohle;
komplexe Metallhydride wie z.B. $LiAlH_4$, $NaBH_4$, $NaBH_3CN$.

Besonders bevorzugt werden folgende Reduktionsmittel eingesetzt:
$NaBH_4$ und $NaBH_3CN$
Das Verfahren wird durchgeführt, indem man die Verbindungen der Formeln II und III in einen Verdünngsmittel in etwa äquivalenten Verhältnis zueinander zusammengibt.

Die Reaktion wird bei Temperaturen von -20 °C bis +100 °C bevorzugt durchgeführt.

Es wird bevorzugt bei Normaldruck gearbeitet.

Als Verdünnungsmittel dienen alle inerten organischen Lösungsmittel. Hierzu gehören insbesondere aliphatische und aromatische Kohlenwasserstoffe wie Pentan, Hexan, Cyclohexan, Petrolether, Ligroin, Benzol, Toluol; chlorierte Kohlenwasserstoffe wie Methylenchlorid, Ethylenchlorid, Chloroform; Ether wie

Diethylether und Glykoldimethylether; Nitrile wie Acetonitril, Propionitril und Benzonitril; Alkohole wie Methanol, Ethanol, n- und i-Propanol.

Bevorzugt sind Alkohole, wobei ohne Isolierung der Zwischenstufen sofort die Reduktion durchgeführt werden kann.

Wie bereits erwähnt, sind die Pyridylhalogenmethylketone der Formel II neu. Sie werden nach dem unter 3 angegebenen Verfahren hergestellt. Dieses läßt sich mit 2-Ethyl3-acetyl-5-carbethoxy-6-methylamino-pyridin und elementarem Brom durch folgendes Formelschema wiedergeben:

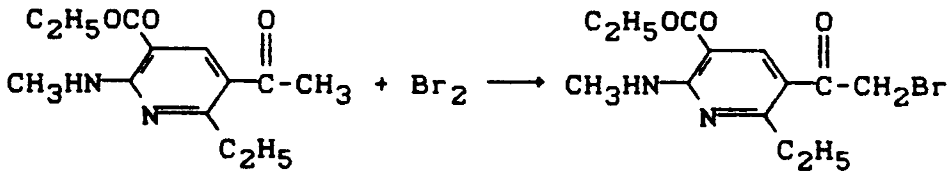

Die zur Durchführung von Verfahren 3 eingesetzten substituierten Pyridylmethylketone der Formel IV sind neu. Ihre Herstellung erfolgt nach dem weiter unten beschriebenen Verfahren.

Bevorzugt sind substituierte Pyridylmethylketone der Formel IV, in welcher $R^1$, $R^2$, $R^3$, $R^4$ die bei den Verbindungen der Formel I genannten bevorzugten Bedeutungen besitzen.

Im einzelnen seien folgende Verbindungen der Formel IV genannt:

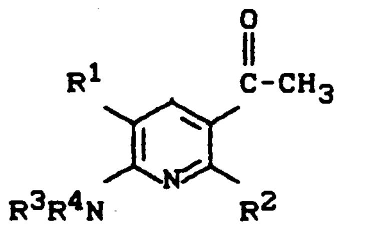

| $R^1$ | $R^2$ | $R^3$ | $R^4$ |
|---|---|---|---|
| C ≡ N | $CH_3$ | H | H |
| C ≡ N | $CH_3$ | $CH_3$ | H |
| C ≡ N | $CH_3$ | $CH_3$ | $CH_3$ |
| C ≡ N | $CH_3$ | $-(CH_2)_4-$ | |
| C ≡ N | Et | H | H |
| 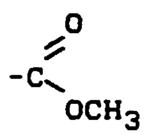 | $CH_3$ | H | H |
| 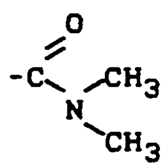 | $CH_3$ | $CH_3$ | $CH_3$ |
| 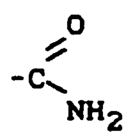 | $CH_3$ | H | H |

Die Halogenierung gemäß Verfahren 3 wird mit geeigneten Halogenierungsmitteln, gegebenenfalls bei erhöhter Temperatur und in Gegenwart von Verdünnungsmitteln, durchgeführt.

Als Halogenierungsmittel seien genannt: elementares Halogen, insbesondere Brom, Kupfer(II)-halogenide, insbesondere $CuBr_2$, Sulfurylchlorid. Die Halogenierungsmittel werden im allgemeinen in etwa stöchiometrischer Menge zu den Verbindungen der Formel IV eingesetzt. Ein bis zu 5-facher, bevorzugt bis 1-5-facher Überschuß Halogenierungsmittel kann vorteilhaft sein.

Die Halogenierung wird zwischen -10 und +150 °C, bevorzugt zwischen 0 und 100 °C, durchgeführt. Weiter bevorzugt ist das Arbeiten bei Siedetemperatur des Verdünnungsmittels.

Es wird bevorzugt bei Normaldruck gearbeitet. Geeignete Verdünnungsmittel sind gegebenenfalls substituierte aliphatische oder aromatische Kohlenwasserstoff wie Pentan, Hexan, Heptan, Cyclohexan, Benzin, Benzol, Ligroin, Petrolether, Toluol, Methylenchlorid, Ethylenchlorid, Chloroform, Tetrachlorkohlenstoff, Chlorbenzol, o-Dichlorbenzol. Für die Durchführung der Halogenierung mit Brom oder $CuBr_2$ seien außerdem genannt Alkohole wie Methanol, Ethanol, Ester wie Essigsäurethylester, Eisessig.

Die Halogenierung mit Brom erfolgt bevorzugt in Gegenwart von äquimolaren Mengen Bromwasserstoffsäure.

Die Aufarbeitung der Reaktionsmischung nach Beendigung der Reaktion erfolgt in an sich bekannter Weise.

Wie bereits erwähnt, sind die Pyridylmethylketone der Formel IV neu. Sie werden nach dem unter 5 angegebenen Verfahren hergestellt. Dieses läßt sich mit 2-Methyl-3-acetyl-5-cyano-6-chlor-pyridin und Pyrrol als Ausgangsverbindungen der Formeln V und VI durch folgendes Formelschema wiedergeben:

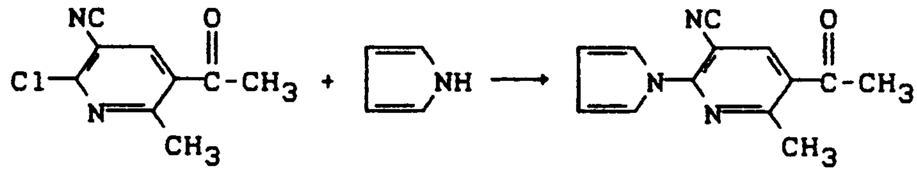

Die zur Durchführung von Verfahren 5 eingesetzten Verbindungen der Formel V sind neu. Ihre Herstellung erfolgt nach dem weiter unten beschriebenen Verfahren. Die Verbindungen der Formel VI sind bekannt. Bevorzugt sind Verbindungen der Formeln V und VI, in denen die Substituenten $R^1$, $R^2$, $R^3$, $R^4$ die bei den Verbindungen der Formel I angegebenen bevorzugten Bedeutungen besitzen und Hal bei Verbindung V für Cl steht.

Im einzelnen seien folgende Verbindungen der Formel V genannt:

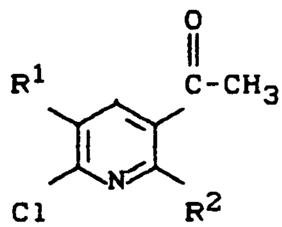

| $R^1$ | $R^2$ |
|---|---|
| C ≡ N | $CH_3$ |
| C ≡ N | Et |
| 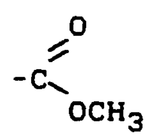 | $CH_3$ |

Im einzelnen seien folgende Amine d genannt:
Ammoniak, Melthylamin, Dimethylamin, Ethylamin, Diethylamin, Methylethylamin, n-Propylamin, i-Propylamin, n-Butylamin, i-Butylamin, tert.-Butylamin, Cyclopentylamin, Cyclohexylamin, Piperidin, Pyrrolidin, Pyrrol, Morpholin.

Die Reaktion wird bei Temperaturen von 0 ° C bis 200 ° C, bevorzugt bei 25 bis 125 ° C, durchgeführt.

Es wird bevorzugt bei Normaldruck gearbeitet. Erfolgt die Umsetzung mit flüchtigen Aminen, wie z.B. Ammoniak, wird bevorzugt unter Druck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Tetramethylensulfon und Hexamethylphosphorsäuretriamid, weiterhin Alkohole wie Methanol, Ethanol, n-und i-Propanol. Die Umsetzung kann auch ohne Verdünnungsmittel erfolgen.

Die Umsetzung kann in Gegenwart von Hilfsbasen wie tertiären organischen Aminen, z.B. Triethylamin, Tripropylamin, Tributylamin, N,N-Dimethylanilin, Pyridin durchgeführt werden.

Die Amine der Formel VI werden bevorzugt im Überschuß (2 bis 10 Mol Amin pro Mol Verbindung der

Formel V) eingesetzt. Arbeitet man mit einer Hilfsbase kann der Überschuß des Amins der Formel V auf 1 bis 3 Mol Amin, bevorzugt 1 bis 1,5 Mol Amin pro Mol Verbindung der Formel V, reduziert werden. Es wurden dann 1 bis 3 Mol Hilfsbase, bevorzugt 1 bis 1,5 Mol Hilfsbase pro Mol Verbindung der Formel V, eingesetzt.

Die Aufarbeitung des Reaktionsgemisches erfolgt in an sich bekannter Weise.

Wie bereits erwähnt, sind die Verbindungen der Formel V neu. Sie werden nach dem unter 7 angegebenen Verfahren hergestellt. Dieses läßt sich mit 2-Methyl-3-acetyl-5-cyano-pyridon(6) als Verbindung der Formel VII und Phosphoroxychlorid als Ausgangsverbindungen durch folgendes Formelschema darstellen:

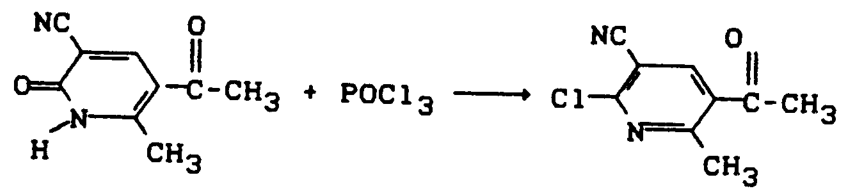

Die zur Durchführung von Verfahren 8 eingesetzten Verbindungen der Formel VII sind bekannt oder lassen sich analog zu bekannten Verfahren herstellen (P. Schenone et al. J. Heterocycl. Chem. 22, S. 1503 (1985); L, Crombie et al., J. Chem. Soc. Perkin Trans 1, S. 677-685 (1979)).

Bevorzugt sind Verbindungen der Formel VII, in der die Substituenten $R^1$ und $R^2$ die bei den Verbindungen der Formel I angegebenen bevorzugten Bedeutungen besitzen.

Im einzelnen seien folgende Verbindungen der Formel VII genannt:

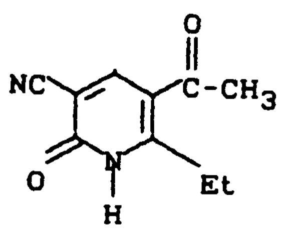

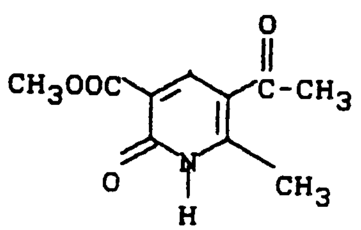

Die Reaktion wird durchgeführt, indem man eine Verbindung der Formel VII mit 1 bis 5 Mol, bevorzugt 1 bis 1,5 Mol des anorganischen Säurechlorids, gegebenenfalls in einem Verdünnungsmittel, behandelt.

Die Umsetzung wird bei Temperaturen von 50 °C bis 150 °C durchgeführt, es wird bevorzugt bei Normaldruck gearbeitet.

Die Umsetzung kann in Gegenwart von Säurebindemitteln erfolgen. Als solche seien bevorzugt tertiäre organische Amine genannt wie z.B. Triethylamin, Tripropylamin, Tributylamin, N,N-Dimethylanilin, Pyridin.

Als Verdünnungsmittel können alle inerten organischen Lösungsmittel verwendet werden. Hierzu zählen insbesondere aliphatische und aromatische, gegebenenfalls halogenierte Kohlenwasserstoffe wie Pentan, Hexan, Cyclohexan, Benzol, Toluol, Melthylenchlorid, Chloroform, Chlorbenzol; Ether wie Diethylether, Tetrahydrofuran, Dioxan, Nitrile, Ester.

Bevorzugt wird ohne Verdünnungsmittel gearbeitet.

Die Aufarbeitung erfolgt in an sich bekannter Weise durch Hydrolysieren des Halogenierungsmittels und Absaugen des Reaktionsproduktes bzw. Abdestillieren des Lösungsmittels.

Die Wirkstoffe eignen sich bei günstiger Warmblütertoxizität als Mittel zur Leistungsförderung bei Zucht- und Nutztieren. Sie dienen dabei zur Förderung und Beschleunigung des Wachstums, der Milch-

und Wollproduktion, sowie zur Verbesserung der Futterverwertung, der Fleischqualität und zur Verschiebung des Fleisch-Fett-Verhältnisses zugunsten von Fleisch.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren. Besonders wertvoll erweisen sich die Wirkstoffe bei Aufzucht und Haltung von Jung- und Masttieren.

Die Anwendung der Wirkstoffe erfolgt direkt oder in Form von für Tiere geeigneten Zubereitungen enteral oder parenteral. Die enterale Anwendung der Wirkstoffe geschieht z.B. oral in Form von Pulvern, Tabletten, Kapseln, Pasten, Tränken, Granulaten, Boli, über oral applizierbare Lösungen, Emulsionen oder Suspensionen sowie über das Futter oder über das Trinkwasser. Die parenterale Anwendung geschieht z.B. in Form der Injektion (intramuskulär, subcutan, intravenös oder durch Implantate).

Besonders hervorgehoben seien Zubereitungen zur Verabreichung über das Futter oder das Trinkwasser. Dabei können die Wirkstoffe dem Futter direkt oder in Form von Prämixen oder Futterkonzentraten zugesetzt werden.

Zum Futter zählen Einzelfuttermittel pflanzlicher Herkunft wie Heu, Rüben, Getreide und Getreidenebenprodukte, Melasse, Silage, Einzelfuttermittel tierischer Herkunft wie Fleisch, Fette, Milchprodukte, Knochenmehl, Fischprodukte, die Einzelfuttermittel wie Vitamine, Proteine, Zucker, Stärke, Mehle, Aminosäuren z.B. DL-Methionin, Salze wie Kalk und Kochsalz. Zum Futter zählen auch Ergänzungs-, Fertig-und Mischfuttermittel. Diese enthalten Einzelfuttermittel in einer Zusammensetzung, die eine ausgewogene Ernährung hinsichtlich der Energie- und Proteinversorgung sowie der Versorgung mit Vitaminen, Mineralsalzen und Spurenelementen sicherstellt.

Prämixe und Futterkonzentrate sind Mischungen des Wirkstoffs mit Trägerstoffen und gegebenenfalls weiteren Hilfsstoffen. Zu den Trägerstoffen zählen alle Einzelfuttermittel oder Gemische derselben.

Die Wirkstoffe können in den Zubereitungen allein oder in Mischung mit anderen leistungsfördernden Wirkstoffen, mineralischen Futtermitteln, Spurenelement-Verbindungen, Vitaminen, stickstoffhaltigen nicht-Protein-Verbindungen, Farbstoffen, Antioxidantien, Aromastoffen, Emulgatoren, Fließhilfsstoffen, Konservierungs- und Preßhilfsstoffen vorliegen.

Andere leistungsfördernde Wirkstoffe sind z.B. Antibiotika wie Tylosin und Virginiamycin.

Mineralische Futtermittel sind z.B. Dicalciumphosphat, Magnesiumoxid, Natriumchlorid.

Spurenelement-Verbindungen sind z.B. Eisenfumarat, Natriumjodid, Kobaltchlorid, Kupfersulfat, Zinkoxid, Selenverbindungen.

Vitamine sind z.B. Vitamin A, Vitamin $D_3$, Vitamin E.

Stickstoffhaltige nicht-Protein-Verbindungen sind z.B. Biuret, Harnstoff.

Farbstoffe sind z.B. Carotinoide wie Canthaxanthin, Zeaxanthin, Capsanthin oder Farbstoffe, die zur Färbung von Lebensmitteln zugelassen sind.

Antioxidantien sind z.B. Ethoxyquin, Butylhydroxy-Toluol, Ascorbinsäure.

Aromastoffe sind z.B. Vanillin.

Emulgatoren sind z.B. Ester der Milchsäure, Lecithin.

Fließhilfsstoffe sind z.B. Natriumstearat, Calciumstearat, Kieselsäuren, Bentonite, Ligninsulfonate.

Konservierungsstoffe sind z.B. Propionsäure, Calciumpropionat, Sorbinsäure, Ascorbinsäure.

Preßhilfsstoffe sind z.B. Ligninsulfonate, Celluloseether.

Die Konzentration der Wirkstoffe im Futter beträgt normalerweise etwa 0,001-500 ppm, bevorzugt 0,1-50 ppm.

Die Konzentration der Wirkstoffe in den Prämixen oder Futterkonzentraten beträgt ca. 0,5 bis 50 Gewichtsprozent, bevorzugt 1 bis 20 Gewichtsprozent.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab.

Die Wirkstoffe können einmalig verabreicht werden. Die Wirkstoffe können aber auch während der

ganzen oder während eines Teils der Wachstums- und Leistungsphase temporär oder kontinuierlich verabreicht werden. Bei kontinuierlicher Verabreichung kann die Anwendung ein-oder mehrmals täglich in regelmäßigen oder unregelmäßigen Abständen erfolgen.

Beispiel für die Zusammensetzung eines Kükenaufzuchtfutters, das erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung der unten angegebenen Zusammensetzung und 2,5 g Wirkstoff-Prämix der unten angegebenen Zusammensetzung ergeben nach sorgfältigem Mischen 1 kg Futter.

In einem kg Vitamin-Mineral-Mischung sind enthalten: 600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $SO_4$ x $H_2O$, 140 mg Zn $SO_4$ x 7 $H_2O$, 100 mg Fe $SO_4$ x 7 $H_2O$ und 20 mg Cu $SO_4$ x5 $H_2O$ in Getreidemehl als Trägerstoff.

1 kg Wirkstoff-Prämix enthalten 100 g Wirkstoff, 900 g Weizenmehl.

Beispiel für die Zusammensetzung eines Schweineaufzuchtfutters, das erfindungsgemäßen Wirkstoff enthält:

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung (Zusammensetzung wie beim Kükenfutter), 30 g Leinkuchenmehl, 30 g Maiskleberfutter, 10 g Sojaöl, 10 g Zuckerrohrmelasse und 2 g Wirkstoff-Prämix ergeben nach sorgfältigem Mischung 1 kg Futter,
1 kg Wirkstoff-Prämix enthält 200 g Wirkstoff, 20 g Pflanzenöl, 780 g Calciumcarbonatpulver.

Beispiel für die Zusammensetzung eines Futters für Rinder, das den erfindungsgemäßen Wirkstoff enthält:

69,95 % Futtergetreideschrot, 10 % gemahlene Maiskolben, 8 % Sojabohnenmehl, 5 % Luzernemehl, 5 % Melasse, 0,6 % Harnstoff, 0,5 % Calciumphosphat, 0,5 % Calciumcarbonat, 0,3 % Kochsalz, 0,15 % Vitamin-Mineral-Mischung und 0,2 % Wirkstoff-Prämix der beim Schweineaufzuchtfutter angegebenen Zusammensetzung. Die Vitamin-Mineral-Mischung enthält pro kg 70.000 i.E. Vitamin A, 70,000 i.E. Vitamin $D_3$, 100 mg Vitamin E, 50 mg Mn $SO_4$ x $H_2O$, 30 mg Zn $SO_4$ x $7H_2O$ in Getreidemehl als Trägerstoff.

Der Wirkstoff-Prämix wird der Vitamin-Mineral-Mischung in der erforderlichen Menge beigemischt und diese anschließend sorgfältig mit den übrigen Bestandteilen vermischt.

Beispiel

Ratten-Fütterungsversuch

Weibliche Laborratten 90-110 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammensetzung des Futters die Vergleichbarkeit der Ergebnisse nicht beeinträchtigen können.

Die Ratten erhalten Wasser ad lib.

Jeweils 12 Ratten bilden eine Versuchsgruppe und werden mit Futter, das mit der gewünschten Menge Wirkstoff versetzt ist gefüttert. Eine Kontrollgruppe erhält Futter ohne Wirkstoff. Die Versuchsgruppen werden so zusammengestellt, daß das durchschnittliche Körpergewicht sowie die Streuung in den Körpergewichten der Ratten in jeder Versuchsgruppe gleich ist, so daß eine Vergleichbarkeit der Versuchsgruppen untereinander gewährleistet ist.

Die Tiere werden vor Versuchsbeginn 2 Tage an die neuen Haltungsbedingungen adaptiert, während dieser Zeit wird Futter ohne Wirkstoffzusatz gegeben. Danach erhalten die Tiere 13 Tage lang Futter das Wirkstoff enthält. Es wird die relative Gewichtszunahme im Verhältnis zur unbehandelten Kontrolle bestimmt.

Es Werden die aus der Tabelle ersichtlichen Ergebnisse erhalten:

Tabelle

| Ratten-Fütterungsversuch | | |
|---|---|---|
| Wirkstoff Bsp.-Nr. | Wirkstoff-konzentration ppm | relative Gewichtszunahme |
| 1 | 25 | 127 |
| 4 | 25 | 119 |
| 6 | 5 | 122 |
| 7 | 5 | 116 |

Herstellungsbeispiele

Beispiel 1

2-N-Isopropylamino-1-(6-amino-5-cyano-2-methyl-3-pyridyl)-ethanol

830 mg (14 mmol) Isopropylamin werden in 50 ml Methanol vorgelegt und 1,2 g (4,7 mmol) 2-Amino-5-bromacetyl-3-cyano-6-methyl-pyridin eingetragen. Es wird auf 50 ° C erwärmt und zwei Stunden bei dieser Temperatur gerührt. Anschließend wird die Suspension auf 0 ° C gekühlt und 400 mg (10,5 mmol) $NaBH_4$ zugegeben.

Nach einer Stunde bei 0 ° C ist die Reaktion vollständig. Zur Aufarbeitung wird im Vakuum eingedampft, der Rückstand zwischen Wasser und Essigester verteilt. Die organische Phase wird abgetrennt, das Produkt in eine 5 %ige $NaH_2PO_4$-Lösung extrahiert und die organische Phase verworfen. Die wäßrige Phase wird mit NaOH alkalisch gestellt und mit $CHCl_3$ extrahiert. Nach Trocknung mit $Na_2SO_4$ wird eingedampft und zur Reinigung aus Essigester umkristallisiert.
Ausbeute: 465,8 mg (42 % der Theorie)
Schmelzpunkt: 158 ° C.

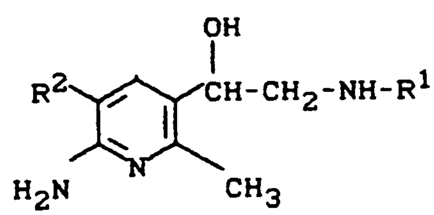

| Bsp.-Nr. | $R^1$ | $R^2$ | Schmp.: |
|---|---|---|---|
| 2 | tert.-Butyl | C ≡ N | 136° C |
| 3 | 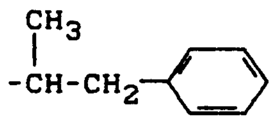 | C ≡ N | 132° C |
| 4 | tert.-Butyl | 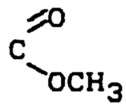 | 148° C |
| 5 | 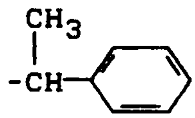 | C≡N | 170° C Diastereomer A<br>147° C Diastereomer B |
| 6 | 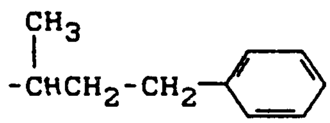 | C≡N | IR: 3400-3200 (breit), 2950, 2200, 1665, 1605, 1495, 1450, 1365, 735, 685 $cm^{-1}$ |
| 7 | 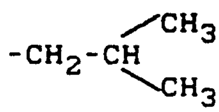 | C≡N | 163° C |
| 8 | 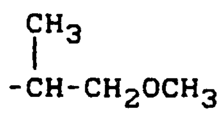 | C≡N | 136° C |
| 9 | 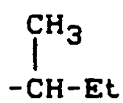 | C≡N | 139° C |
| 10 | 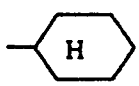 | C≡N | 149° C |
| 11 | 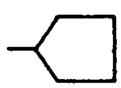 | C≡N | 170° C |
| 12 | 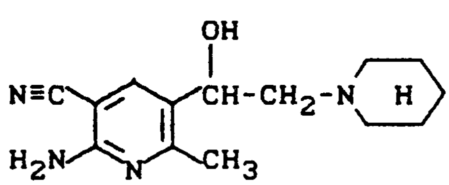 | | 144° C |

Beispiel 13

Beispiel für Verfahren 4

2-Amino-5-bromacetyl-3-cyano-6-methyl-pyridin

2,7 g (15,4 mmol) 5-Acetyl-2-amino-3-cyano-6-methyl-pyridin werden in 110 ml Eisessig suspendiert und mit 1,9 ml (16,2 mmol) 48 %iger HBr versetzt. Dann werden 5,2 g (32,4 mmol) Brom zugegeben und fünf Stunden bei 45 ° C gerührt. Nach Abkühlung wird abgesaugt, der Filterkuchen mit $NaHCO_3$-Lösung verrührt, abgesaugt und getrocknet. Zur Reinigung wird aus Ethanol umkristallisiert.
Ausbeute: 2,3 g (59 % der Theorie)
Schmelzpunkt: 209 ° C (Zersetzung).

Beispiel 14

Beispiel für Verfahren 6

3-Acetyl-5-cyano-2-methyl-6-morpholino-pyridin

9,5 g (48,8 mmol) 3-Acetyl-6-chlor-5-cyano-2-methyl-pyridin werden in 180 ml Toluol gelöst, 9,5 g (0,109 mol) Morpholin zugegeben und das Ganze zwei Stunden bei 90 ° C gerührt. Zur Aufarbeitung wird eingedampft, der Rückstand in Wasser suspendiert, abgesaugt und sorgfältig mit Wasser gewaschen. Zur Reinigung wird aus Methanol/Wasser umkristallisiert.
Ausbeute: 11,1 g (92,8 % der Theorie)
Schmelzpunkt: 126 ° C.

Weitere Beispiele für Verfahren 6

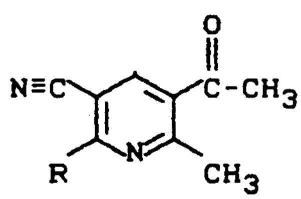

| Bsp.-Nr. | R | Schmp.: |
|---|---|---|
| 15 | $-NH-i-C_3H_7$ | 105° C |
| 16 | 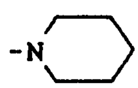 | 91° C |
| 17 | 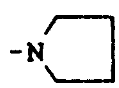 | 134° C |

Analog werden erhalten:

Beispiel 18

3-Acetyl-5-cyano-2-methyl-6-methylaminopyridin.
Schmelzpunkt: 239 ° C.

Beispiel 19

5-Acetyl-2-amino-3-cyano-6-methyl-pyridin

40 g (0,21 mol) 5-Acetyl-2-chlor-3-cyano-6-methyl-pyridin werden in 800 ml Tetrahydrofuran gelöst, 200 ml konzentrierte wäßrige $NH_3$-Lösung zugegeben und in einem Rührautoklaven zwei Stunden auf 80 ° C erhitzt. Nach dem Abkühlen wird eingeengt, abfiltriert, der Filterkuchen in Acetonitril ausgekocht und

abgesaugt.
Ausbeute: 27,2 g (75,5 % der Theorie)
Schmelzpunkt: 219 ° C, gelbe Plättchen.

Beispiel für Verfahren 8

Beispiel 20

3-Acetyl-5-cyano-6-chlor-2-methyl-pyridin

35 g (0,2 mol) 5-Acetyl-3-cyano-6-methyl-pyridon (2)*werden in 400 ml Chlorbenzol suspendiert, 24,2 g (0,2 mol) N,N-Dimethylanilin zugegeben und 37 g (0,24 mol) $POCl_3$ zugetropft. Anschließend wird drei Stunden auf 100 ° C erhitzt. Nach dem Abkühlen wird vorsichtig auf Wasser gegossen, mit verdünnter Natronlauge neutralisiert, die organische Phase abgetrennt und die wäßrige Phase nochmals mit $CHCl_3$ nachextrahiert. Die vereinigten Extrakte werden mit $Na_2SO_4$ getrocknet und eingedampft. Der Rückstand wird durch Filtration über Kieselgel, mit $CH_2Cl_2$/Essigester als Laufmittel, gereinigt.
Ausbeute: 26,7 g (68,5 % der Theorie)
Schmelzpunkt: 98 ° C.

## Patentansprüche

**1.** Substituierte Pyridylethanolamine der Formel I,

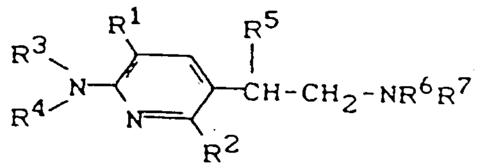

I

in welcher

| | |
|---|---|
| $R^1$ | für CN, $-COOR^8$, $-CONR^9R^{10}$ steht, |
| $R^2$ | für $C_{1-4}$-Alkyl steht, |
| $R^3$ | für Wasserstoff oder $C_{1-4}$-Alkyl steht, |
| $R^4$ | für Wasserstoff oder $C_{1-4}$-Alkyl steht oder $R^3$ und $R^4$ gemeinsam mit dem Stickstoffatom, an das sie gebunden sind, für einen gegebenenfalls durch Halogen oder $C_{1-4}$-Alkyl substituierten Heterocyclus aus der Reihe Pyrrolidin, Pyrrol, Piperidin, Morpholin stehen, |
| $R^5$ | für OH, $C_{1-6}$-Alkoxy oder Acyloxy steht und Acyloxy für Oxycarbonyl-$C_{1-6}$-alkyl, Oxycarbonylphenyl, Oxysulfonyl-$C_{1-6}$-alkyl, Oxysulfonylphenyl steht, |
| $R^6$ | für Wasserstoff oder $C_{1-6}$-Alkyl steht, |
| $R^7$ | für Wasserstoff, gegebenenfalls durch 1 bis 5 Halogenatome substituiertes $C_{1-6}$-Alkyl, Cycloalkyl mit bis zu 6 C-Atomen, $C_{1-6}$-Alkylphenyl, das gegebenenfalls durch Halogen, $C_{1-4}$-Alkyl,Hydroxy, $C_{1-4}$-Alkoxy, gegebenenfalls halogensubstituiertes Methylendioxy oder Ethylendioxy substituiert sein kann; $C_{1-6}$-Alkylphenoxy, das gegebenenfalls durch Halogen, $C_{1-4}$-Alkyl, Hydroxy, $C_{1-4}$-Alkoxy, gegebenenfalls halogensubstituiertes Methylendioxy oder Ethylendioxy substituiert sein kann, steht, |
| $R^6$ und $R^7$ | können gemeinsam mit dem Stickstoffatom, an das sie gebunden sind, für einen 5- bis 6-gliedrigen gesättigten oder ungesättigten heterocyclischen Rest stehen, der gegebenenfalls noch weitere Heteroatome aus der Reihe N, O, S enthalten kann, |
| $R^8$ | für $C_{1-6}$-Alkyl, |
| $R^9$ und $R^{10}$ | unabhängig voneinander für Wasserstoff oder $C_{1-4}$-Alkyl stehen, |

sowie ihre physiologisch verträglichen Salze und ihre N-Oxide.

* Herstellung L. Mosti, P. Schenone und G. Mennozzi, J. Heterocyc. Chem. 22, 1503 (1985).

**2.** Verfahren zur Herstellung der substituierten Pyridylethanolamine der Formel I

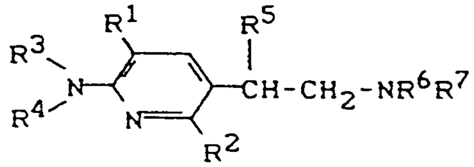

I

in welcher

| | |
|---|---|
| $R^1$ | für CN, $-COOR^8$, $-CONR^9R^{10}$ steht, |
| $R^2$ | für $C_{1-4}$-Alkyl steht, |
| $R^3$ | für Wasserstoff oder $C_{1-4}$-Alkyl steht, |
| $R^4$ | für Wasserstoff oder $C_{1-4}$-Alkyl steht oder $R^3$ und $R^4$ gemeinsam mit dem Stickstoffatom, an das sie gebunden sind, für einen gegebenenfalls durch Halogen oder $C_{1-4}$-Alkyl substituierten Heterocyclus aus der Reihe Pyrrolidin, Pyrrol, Piperidin, Morpholin stehen, |
| $R^5$ | für OH, $C_{1-6}$-Alkoxy oder Acyloxy steht und Acyloxy für Oxycarbonyl-$C_{1-6}$-alkyl, Oxycarbonylphenyl, Oxysulfonyl$C_{1-6}$-alkyl, Oxysulfonyl-$C_{1-6}$-alkyl, Oxysulfonylphenyl steht, |
| $R^6$ | für Wasserstoff oder $C_{1-6}$-Alkyl steht, |
| $R^7$ | für Wasserstoff, gegebenenfalls durch 1 bis 5 Halogenatome substituiertes $C_{1-6}$-Alkyl, Cycloalkyl mit bis zu 6 C-Atomen, $C_{1-6}$-Alkylphenyl, das gegebenenfalls durch Halogen, $C_{1-4}$-Alkyl, Hydroxy, $C_{1-4}$-Alkoxy, gegebenenfalls halogensubstituiertes Methylendioxy oder Ethylendioxy substituiert sein kann; $C_{1-6}$-Alkylphenoxy, das gegebenenfalls durch Halogen, $C_{1-4}$-Alkyl, Hydroxy, $C_{1-4}$-Alkoxy, gegebenenfalls halogensubstituiertes Methylendioxy oder Ethylendioxy substituiert sein kann, steht, |
| $R^6$ und $R^7$ | können gemeinsam mit dem Stickstoffatom, an das sie gebunden sind, für einen 5- bis 6-gliedrigen gesättigten oder ungesättigten heterocyclischen Rest stehen, der gegebenenfalls noch weitere Heteroatome aus der Reihe N, O, S enthalten kann, |
| $R^8$ | für $C_{1-6}$-Alkyl, |
| $R^9$ und $R^{10}$ | unabhängig voneinander für Wasserstoff oder $C_{1-4}$-Alkyl stehen. |

sowie ihre physiologisch verträglichen Salze und ihre N-Oxide,

dadurch gekennzeichnet, daß man substituierte Pyridylhalogenmethylketone der Formel II,

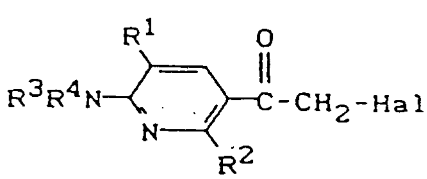

II

in welcher

$R^1$, $R^2$, $R^3$, $R^4$ die oben angegebenen Bedeutungen haben,

Hal für Halogen steht,

mit Aminen der Formel III,

$HNR^6R^7$ III

in welcher

$R^6$ und $R^7$ die oben angegebene Bedeutung haben,

umsetzt und anschließend die Carbonylgruppe reduziert und danach gegebenenfalls acyliert oder alkyliert.

**3.** Substituierte Pyridylhalogenmethylketone der Formel

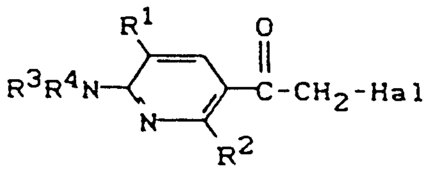

II

in welcher

| | |
|---|---|
| $R^1$ | für CN, -$COOR^8$, -$CONR^9R^{10}$ steht, |
| $R^2$ | für $C_{1-4}$-Alkyl steht, |
| $R^3$ | für Wasserstoff oder $C_{1-4}$-Alkyl steht, |
| $R^4$ | für Wasserstoff oder $C_{1-4}$-Alkyl steht oder $R^3$ und $R^4$ gemeinsam mit dem Stickstoffatom, an das sie gebunden sind, für einen gegebenenfalls durch Halogen oder $C_{1-4}$-Alkyl substituierten Heterocyclus aus der Reihe Pyrrolidin, Pyrrol, Piperidin, Morpholin stehen, |
| $R^8$ | für $C_{1-6}$-Alkyl, |
| $R^9$ und $R^{10}$ | unabhängig voneinander für Wasserstoff oder $C_{1-4}$-Alkyl sthen, |

Hal für Halogen steht,

sowie ihre physiologisch verträglichen Salze und ihre N-Oxide.

**4.** Verfahren zur Herstellung der Pyridylhalogenmethylketone der Formel II,

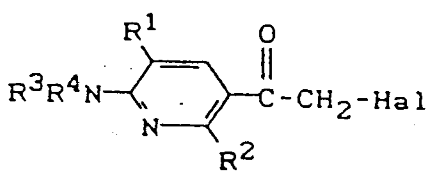

II

in welcher

$R^1$, $R^2$, $R^3$, $R^4$, Hal die in Anspruch 3 angegebene Bedeutung haben,

dadurch gekennzeichnet, daß man substituierte Pyridylmethylketone der Formel IV,

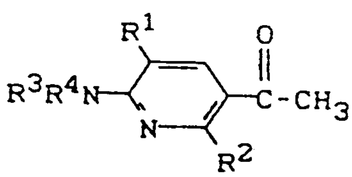

IV

in welcher

$R^1$, $R^2$, $R^3$, $R^4$ die oben angegebene Bedeutung haben,

halogeniert.

**5.** Substituierte Pyridylmethylketone der Formel IV,

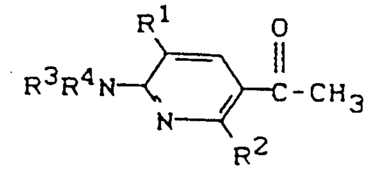

IV

in welcher

| | |
|---|---|
| $R^1$ | für CN, $-COOR^8$, $-CONR^9R^{10}$ steht, |
| $R^2$ | für $C_{1-4}$-Alkyl steht, |
| $R^3$ | für Wasserstoff oder $C_{1-4}$-Alkyl steht, |
| $R^4$ | für Wasserstoff oder $C_{1-4}$-Alkyl steht oder $R^3$ und $R^4$ gemeinsam mit dem Stickstoffatom, an das sie gebunden sind, für einen gegebenenfalls durch Halogen oder $C_{1-4}$-Alkyl substituierten Heterocyclus aus der Reihe Pyrrolidin, Pyrrol, Piperidin, Morpholin stehen, |
| $R^8$ | für $C_{1-6}$-Alkyl, |
| $R^9$ und $R^{10}$ | unabhängig voneinander für Wasserstoff oder $C_{1-4}$-Alkyl stehen, |

sowie ihre physiologisch verträglichen Salze und ihre N-Oxide.

**6.** Verfahren zur Herstellung der substituierten Pyridylmethylketone der Formel IV,

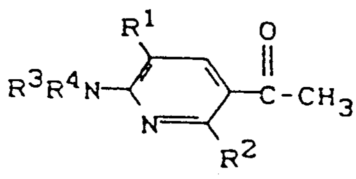

IV

in welcher

$R^1$, $R^2$, $R^3$, $R^4$ die in Anspruch 5 angegebene Bedeutung haben,

dadurch gekennzeichnet, daß man Pyridylmethylketone der Formel V,

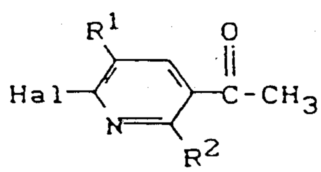

V

in welcher

$R^1$ und $R^2$ die oben angegebene Bedeutung haben,

Hal für Halogen steht,

mit Aminen der Formel VI,

$H-NR^3R^4$ VI

in welcher

$R^3$ und $R^4$ die oben angegebene Bedeutung haben,

umsetzt.

**7.** Pyridylmethylketone der Formel V,

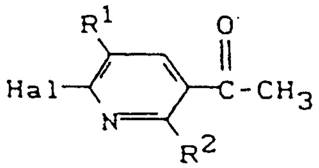

V

in welcher

| | |
|---|---|
| $R^1$ | für CN, -$COOR^8$, -$CONR^9R^{10}$ steht, |
| $R^2$ | für $C_{1-4}$-Alkyl steht, |
| $R^8$ | für $C_{1-6}$-Alkyl, |
| $R^9$ und $R^{10}$ | unabhängig voneinander für Wasserstoff oder $C_{1-4}$-Alkyl stehen, |

Hal für Halogen steht,

sowie ihre physiologisch verträglichen Salze und ihre N-Oxide.

**8.** Verfahren zur Herstellung der Pyridylmethylketone der Formel V,

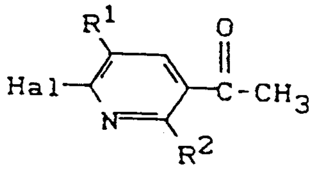

V

in welcher

$R^1$, $R^2$, Hal die in Anspruch 7 angegebene Bedeutungen haben,

dadurch gekennzeichnet, daß man Verbindungen der Formel VII,

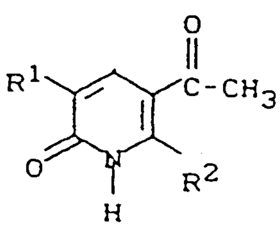

VII

in welcher

$R^1$ und $R^2$ die oben angegebene Bedeutung haben,

mit Phosphoroxychlorid, gegebenenfalls in Gegenwart von Säurebindemitteln, umsetzt.

**9.** Mittel zur Leistungsförderung von Tieren, gekennzeichnet durch einen Gehalt an substituierten Pyridylethanolaminen der Formel I gemäß Anspruch 1.

**10.** Verwendung von substituierten Pyridylethanolaminen der Formel I gemäß Anspruch 1 zur Leistungsförderung von Tieren.

**11.** Verbindung der Formel

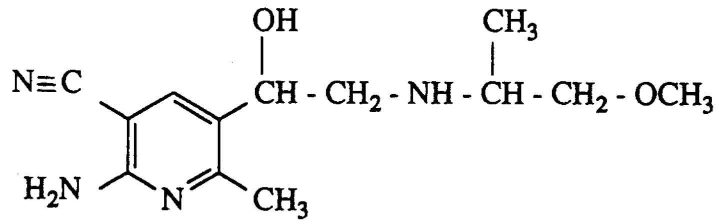

**Claims**

**1.** Substituted pyridylethanolamines of the formula I

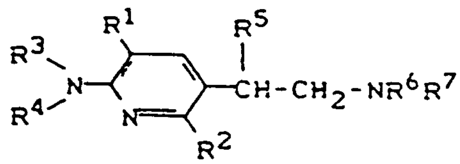

I

in which

| | |
|---|---|
| $R^1$ | represents CN, $-COOR^8$ or $-CONR^9R^{10}$, |
| $R^2$ | represents $C_{1-4}$-alkyl, |
| $R^3$ | represents hydrogen or $C_{1-4}$-alkyl, |
| $R^4$ | represents hydrogen or $C_{1-4}$ alkyl, or $R^3$ and $R^4$, together with the nitrogen atom to which they are bonded, represent an optionally halogen or $C_{1-4}$-alkyl-substituted heterocycle from the series comprising pyrrolidine, pyrrole, piperidine and morpholine, |
| $R^5$ | represents OH, $C_{1-6}$-alkoxy or acyloxy, and acyloxy represents oxycarbonyl-$C_{1-6}$-alkyl, oxycarbonylphenyl, oxysulphonyl-$C_{1-6}$-alkyl or oxysulphonylphenyl, |
| $R^6$ | represents hydrogen or $C_{1-6}$-alkyl, |
| $R^7$ | represents hydrogen, $C_{1-6}$-alkyl which is optionally substituted by 1 to 5 halogen atoms, or cycloalkyl having up to 6 C atoms, $C_{1-6}$-alkylphenyl which can optionally be substituted by halogen, $C_4$-alkyl, hydroxyl, $C_{1-4}$-alkoxy, optionally halogen-substituted methylenedioxy or ethylenedioxy; $C_{1-8}$-alkylphenoxy which can optionally be substituted by halogen, $C_{1-4}$-alkyl, hydroxyl, $C_{1-4}$-alkoxy, optionally halogen substituted methylenedioxy or ethylenedioxy, |
| $R^6$ and $R^7$ | can, together with the nitrogen atom to which they are bonded, represent a 5- to 6-membered saturated or unsaturated heterocyclic radical, which can optionally also contain further hetero atoms from the series comprising N, O and S, |
| $R^8$ | represents $C_{1-6}$-alkyl, and |
| $R^9$ and $R^{10}$, | independently of one another, represent hydrogen or $C_{1-4}$-alkyl, |

and their physiologically tolerated salts and their N-oxides.

**2.** Process for the preparation of the substituted pyridylethanolamines of the formula I

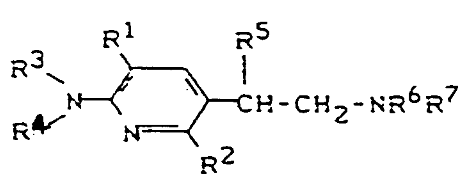

I

in which

- $R^1$ represents CN, $-COOR^8$ or $-CONR^8R^{10}$,
- $R^2$ represents $C_{1-4}$-alkyl,
- $R^3$ represents hydrogen or $C_{1-4}$-alkyl,
- $R^4$ represents hydrogen or $C_{1-4}$ alkyl, or $R^3$ and $R^4$, together with the nitrogen atom to which they are bonded, represent an optionally halogen or $C_{1-4}$-alkyl-substituted heterocycle from the series comprising pyrrolidine, pyrrole, piperidine and morpholine,
- $R^5$ represents OH, $C_{1-6}$-alkoxy or acyloxy, and acyloxy represents oxycarbonyl-$C_{1-6}$alkyl, oxycarbonylphenyl, oxysulphonyl-$C_{1-6}$alkyl or oxysulphonylphenyl,
- $R^6$ represents hydrogen or $C_{1-6}$-alkyl,
- $R^7$ represents hydrogen, $C_{1-6}$-alkyl which is optionally substituted by 1 to 5 halogen atoms, or cycloalkyl having up to 6 C atoms, $C_{1-6}$-alkylphenyl which can optionally be substituted by halogen, $C_{1-4}$-alkyl, hydroxyl, $C_{1-4}$-alkoxy, optionally halogen-substituted methylenedioxy or ethylenedioxy; $C_{1-6}$-alkylphenoxy which can optionally be substituted by halogen, $C_{1-4}$-alkyl, hydroxyl, $C_{1-4}$-alkoxy, optionally halogen substituted methylenedioxy or ethylenedioxy,
- $R^6$ and $R^7$ can, together with the nitrogen atom to which they are bonded, represent a 5- to 6-membered saturated or unsaturated heterocyclic radical, which can optionally also contain further hetero atoms from the series comprising N, O and S,
- $R^8$ represents $C_{1-6}$-alkyl, and
- $R^9$ and $R^{10}$, independently of one another, represent hydrogen or $C_{1-4}$-alkyl,

and their physiologically tolerated salts and their N-oxides

characterized in that substituted pyridyl halogenomethyl ketones of the formula II

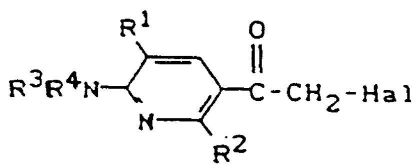

II

in which

$R^1$, $R^2$, $R^3$ and $R^4$ have the abovementioned meanings, and

Hal represents halogen,

are reacted with amines of the formula III

$HNR^6R^7$ III

in which

$R^6$ and $R^7$ have the abovementioned meaning,

and then the carbonyl group is reduced and thereafter, where appropriate, acylated or alkylated.

**3.** Substituted pyridyl halogenomethyl ketones of the formula II

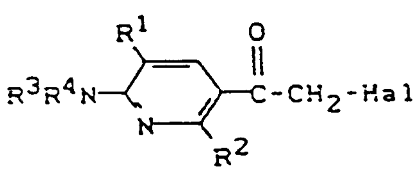

II

in which

- $R^1$ represents CN, - $COOR^8$ or $-CONR^9R^{10}$,
- $R^2$ represents $C_{1-4}$-alkyl,
- $R^3$ represents hydrogen or $C_{1-4}$-alkyl,
- $R^4$ represents hydrogen or $C_{1-4}$alkyl, or $R^3$ and $R^4$ represent, together with the nitrogen atom, to which they are bonded, an optionally halogen- or $C_{1-4}$-alkyl-substituted heterocycle from the series comprising pyrrolidine, pyrrole, piperidine and morpholine,
- $R^8$ represents $C_{1-6}$-alkyl,
- $R^9$ and $R^{10}$, independently of one another, represent hydrogen or $C_{1-4}$-alkyl, and

Hal represents halogen,

and their physiologically tolerated salts and their N-oxides.

**4.** Process for the preparation of the pyridyl halogenomethyl ketones of the formula II

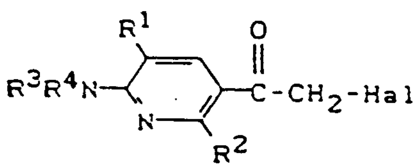

II

in which

$R^1$, $R^2$, $R^3$ $R^4$ and Hal have the meaning indicated in Claim 3,

characterized in that substituted pyridyl methyl ketones of the formula IV

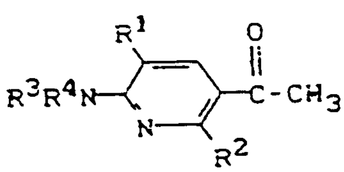

IV

in which

$R^1$, $R^2$, $R^3$ and $R^4$ have the abovementioned meaning,

are halogenated.

**5.** Substituted pyridyl methyl ketones of the formula IV

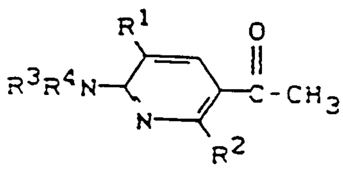

IV

in which

- $R^1$ represents CN, $-COOR^8$ or $-CONR^9R^{10}$,
- $R^2$ represents $C_{1-4}$-alkyl,
- $R^3$ represents hydrogen or $C_{1-4}$-alkyl,
- $R^4$ represents hydrogen or $C_{1-4}$-alkyl, or $R^3$ and $R^4$ represent, together with the

nitrogen atom to which they are bonded, an optionally halogen- or $C_{1-4}$-alkyl-substituted heterocycle from the series comprising pyrrolidine, pyrrole, piperidine and morpholine,

$R^8$ represents $C_{1-6}$-alkyl, and

$R^9$ and $R^{10}$, independently of one another, represent hydrogen or $C_{1-4}$-alkyl,

and their physiologically tolerated salts and their N-oxides.

**6.** Process for the preparation of the substituted pyridyl methyl ketones of the formula IV

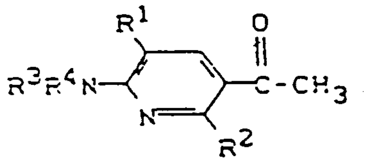

IV

in which

$R^1$, $R^2$, $R^3$ $R^4$ have the meaning indicated in Claim 5, characterized in that pyridyl methyl ketones of the formula V

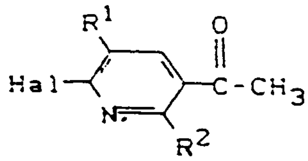

V

in which

$R^1$ and $R^2$ have the abovementioned meaning,

Hal represents halogen,

are reacted with amines of the formula VI

$H\text{-}NR^3R^4$ VI

in which

$R^3$ and $R^4$ have the abovementioned meaning.

**7.** Pyridyl methyl ketones of the formula V

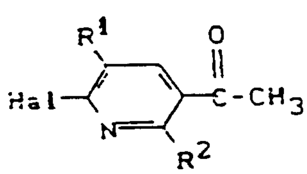

V

in which

$R^1$ represents CN, $-COOR^8$ or $-CONR^9R^{10}$,

$R^2$ represents $C_{1-4}$-alkyl

$R^8$ represents $C_{1-6}$-alkyl,

$R^9$ and $R^{10}$, independently of one another, represent hydrogen or $C_{1-4}$-alkyl, and

Hal represents halogen,

and their physiologically tolerated salts and their N-oxides.

**8.** Process for the preparation of the pyridyl methyl ketones of the formula V

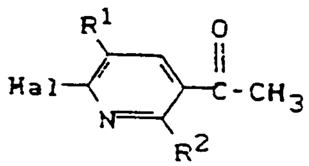

V

in which

in which

$R^1$, $R^2$ and Hal have the meanings indicated in Claim 7,

characterized in that compounds of the formula VII

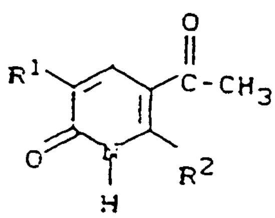

VII

in which

$R^1$ and $R^2$ have the abovementioned meaning, are reacted with phosphorous oxychloride, where appropriate in the presence of acid-binding agents.

**9.** Agent for promoting production of livestock, characterized by containing substituted pyridylethanolamines of the formula I according to Claim 1.

**10.** Use of substituted pyridylethanolamines of the formula I according to Claim 1 for promoting production in livestock.

**11.** Compound of the formula

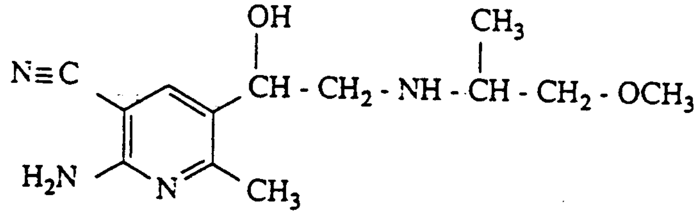

## Revendications

**1.** Pyridyléthanolamines substituées de formule I,

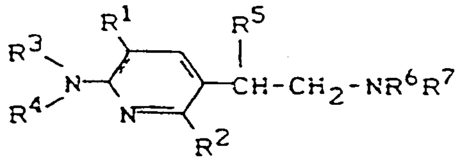

I

dans laquelle

$R^1$ est un groupe CN, $-COOR^8$, $-CONR^9R^{10}$,

$R^2$ est un groupe alkyle en $C_1$ à $C_4$,

$R^3$ est l'hydrogène ou un groupe alkyle en $C_1$ à $C_4$,

$R^4$ est l'hydrogène ou un groupe alkyle en $C_1$ à $C_4$, ou bien $R^3$ et $R^4$ forment conjointement avec l'atome d'azote auquel ils sont liés un hétérocycle éventuellement substitué par un halogène ou par un radical alkyle en $C_1$ à $C_4$, de la série de la pyrrolidine, du pyrrole, de la pipéridine, de la morpholine,

$R^5$ est un groupe OH, alkoxy en $C_1$ à $C_6$ ou acyloxy et le groupe acyloxy représente un groupe oxycarbonyl-(alkyle en $C_1$ à $C_6$), oxycarbonylphényle, oxysulfonyl-(alkyle en $C_1$ à $C_6$), oxysulfonylphényle,

$R^6$ est l'hydrogène ou un groupe alkyle en $C_1$ à $C_6$,

$R^7$ est l'hydrogène, un groupe alkyle en $C_1$ à $C_6$ éventuellement substitué par 1 à 5 atomes d'halogènes, un groupe cycloalkyle ayant jusqu'à 6 atomes de carbone, un groupe (alkyle en $C_1$ à $C_6$)-phényle qui peut être substitué le cas échéant par un halogène, un radical alkyle en $C_1$ à $C_4$, hydroxy, alkoxy en $C_1$ à $C_4$, méthylènedioxy ou éthylènedioxy portant éventuellement un substituant halogéno ; un groupe (alkyle en $C_1$ à $C_6$)phénoxy qui peut être substitué le cas échéant par un halogène, un radical alkyle en $C_1$ à $C_4$, hydroxy, alkoxy en $C_1$ à $C_4$, méthylènedioxy ou éthylènedioxy portant éventuellement des substituants halogéno,

$R^6$ et $R^7$ peuvent former conjointement avec l'atome d'azote auquel ils sont liés un reste hétérocyclique saturé ou non saturé, pentagonal ou hexagonal qui peut comporter, le cas échéant, encore d'autres hétéro-atomes de la série N, O, S,

$R^8$ est un groupe alkyle en $C_1$ à $C_6$,

$R^9$ et $R^{10}$ représentent, indépendamment l'un de l'autre, de l'hydrogène ou un groupe alkyle en $C_1$ à $C_4$,

ainsi que leurs sels et leurs N-oxydes acceptables du point de vue physiologique.

**2.** Procédé de production des pyridyléthanolamines substituées de formule I

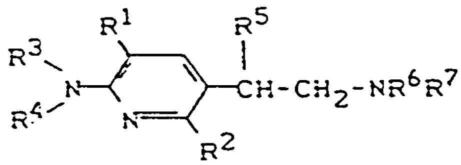

I

dans laquelle

$R^1$ est un groupe CN, $-COOR^8$, $-CONR^9R^{10}$,

$R^2$ est un groupe alkyle en $C_1$ à $C_4$,

$R^3$ est de l'hydrogène ou un groupe alkyle en $C_1$ à $C_4$,

$R^4$ est l'hydrogène ou un groupe alkyle en $C_1$ à $C_4$, ou bien $R^3$ et $R^4$ forment conjointement avec l'atome d'azote auquel ils sont liés un hétérocycle éventuellement substitué par un halogène ou un radical alkyle en $C_1$ à $C_4$, de la série de pyrrolidine, pyrrole, pipéridine, morpholine,

$R^5$ est un groupe OH, alkoxy en $C_1$ à $C_6$ ou acyloxy et acyloxy désigne un groupe oxycarbonyl-(alkyle en $C_1$ à $C_6$), oxycarbonylphényle, oxysulfonyl(alkyle en $C_1$ à $C_6$), oxysulfonyl-(alkyle en $C_1$ à $C_6$), oxysulfonylphényle,

$R^6$ est de l'hydrogène ou un groupe alkyle en $C_1$ à $C_6$,

| | |
|---|---|
| $R^7$ | est de l'hydrogène, un groupe alkyle en $c_1$ à $C_6$ éventuellement substitué par 1 à 5 atomes d'halogènes, un groupe cycloalkyle ayant jusqu'à 6 atomes de carbone, un groupe (alkyle en $C_1$ à $C_6$)-phényle qui peut être substitué, le cas échéant par un halogène, un radical alkyle en $C_1$ à $C_4$, hydroxy, alkoxy en $C_1$ à $C_4$, méthylènedioxy ou éthylènedioxy portant facultativement des substituant halogéno ; un groupe (alkyle en $C_1$ à $C_6$)phénoxy qui peut être substitué, le cas échéant par un halogène, un radical alkyle en $C_1$ à $C_4$, hydroxy, alkoxy en $C_1$ à $C_4$, méthylènedioxy ou éthylènedioxy portant facultativement des substituants halogéno, |
| $R^6$ et $R^7$ | peuvent former conjointement avec l'atome d'azote auquel ils sont liés un reste hétérocyclique saturé ou non saturé, pentagonal ou hexagonal qui peut contenir, le cas échéant, encore d'autres hétéro-atomes de la série N, O, S, |
| $R^8$ | est un groupe alkyle en $C_1$ à $C_6$, |
| $R^9$ et $R^{10}$ | représentent, indépendamment l'un de l'autre, de l'hydrogène ou un groupe alkyle en $C_1$ à $C_4$, |

ainsi que leurs sels et leurs N-oxydes acceptables du point de vue physiologique,

caractérisé en ce qu'on fait réagir des pyridylhalogénométhylcétones substituées de formule II,

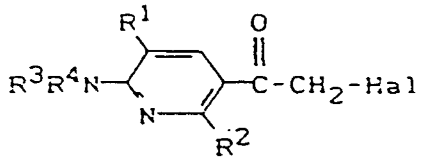

II

dans laquelle

$R^1$, $R^2$, $R^3$, $R^4$ ont les définitions indiquées ci-dessus,

Hal représente un halogène,

avec des amines de formule III,

$HNR^6R^7$ III

dans laquelle

$R^6$ et $R^7$ ont la définition indiquée ci-dessus,

puis on réduit le groupe carbonyle, après quoi on l'acyle ou on l'alkyle, le cas échéant.

**3.** Pyridylhalogénométhylcétones substituées de formule II,

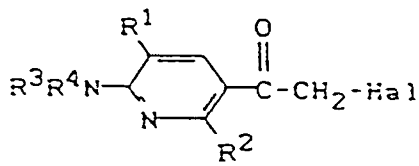

II

dans laquelle

| | |
|---|---|
| $R^1$ | est un groupe CN, $-COOR^8$, $-CONR^9R^{10}$, |
| $R^2$ | est un groupe alkyle en $C_1$ à $C_4$, |
| $R^3$ | est de l'hydrogène ou un groupe alkyle en $C_1$ à $C_4$, |
| $R^4$ | est de l'hydrogène ou un groupe alkyle en $C_1$ à $C_4$, ou bien $R^3$ et $R^4$ forment conjointement avec l'atome d'azote auquel ils sont liés un hétérocycle éventuellement substitué par un halogène ou par un radical alkyle en $C_1$ à $C_4$, de la série pyrrolidine, pyrrole, pipéridine, morpholine, |
| $R^8$ | est un groupe alkyle en $C_1$ à $C_6$, |
| $R^9$ et $R^{10}$ | représentent, indépendamment l'un de l'autre, l'hydrogène ou un groupe alkyle en $C_1$ à $C_4$, |
| Hal | est un halogène, |

ainsi que leurs sels et leurs N-oxydes acceptables du point de vue physiologique.

**4.** Procédé de production de pyridylhalogénométhylcétones de formule II,

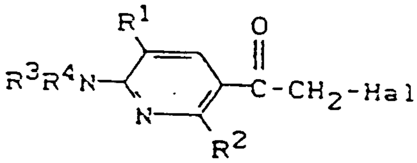

II

dans laquelle
$R^1$, $R^2$, $R^3$, $R^4$, Hal ont la définition indiquée dans la revendication 3,
caractérisé en ce qu'on halogène des pyridylméthylcétones substituées de formule IV,

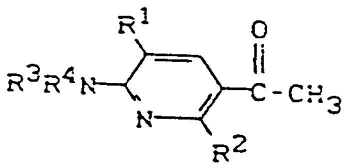

IV

dans laquelle
$R^1$, $R^2$, $R^3$, $R^4$ ont la définition indiquée ci-dessus.

**5.** Pyridylméthylcétones substituées de formule IV,

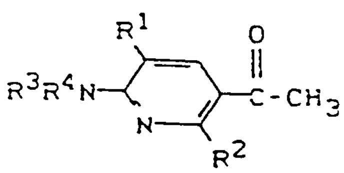

IV

dans laquelle

| | |
|---|---|
| $R^1$ | est un groupe CN, $-COOR^8$, $-CONR^9R^{10}$, |
| $R^2$ | est un groupe alkyle en $C_1$ à $C_4$, |
| $R^3$ | est de l'hydrogène ou un groupe alkyle en $C_1$ à $C_4$, |
| $R^4$ | est de l'hydrogène ou un groupe alkyle en $C_1$ à $C_4$, ou bien $R^3$ et $A^4$ forment conjointement avec l'atome d'azote auquel ils sont liés un hétérocycle éventuellement substitué par un halogène ou un radical alkyle en $C_1$ à $C_4$, de la série pyrrolidine, pyrrole, pipéridine, morpholine, |
| $R^8$ | est un groupe alkyle en $C_1$ à $C_6$, |
| $R^9$ et $R^{10}$ | représentent, indépendamment l'un de l'autre, l'hydrogène ou un groupe alkyle en $C_1$ à $C_4$, |

ainsi que leurs sels et leurs N-oxydes acceptables du point de vue physiologique.

**6.** Procédé de production des pyridylméthylcétones substituées de formule IV,

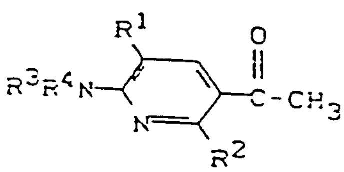

IV

dans laquelle

$R^1$, $R^2$, $R^3$, $R^4$ ont la définition indiquée dans la revendication 5,
caractérisé en ce qu'on fait réagir des pyridylméthylcétones de formule V,

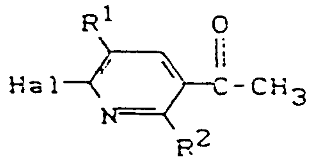

V

dans laquelle
$R^1$ et $R^2$ ont la définition indiquée ci-dessus,
Hal représente un halogène,
avec des amines de formule VI,

$H-NR^3R^4$ VI

dans laquelle
$R^3$ et $R^4$ ont la définition indiquée ci-dessus.

**7.** Pyridylméthylcétones de formule V,

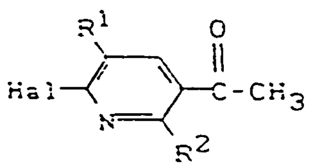

V

dans laquelle
$R^1$ est un groupe CN, $-COOR^8$, $-CONR^9R^{10}$,
$R^2$ est un groupe alkyle en $C_1$ à $C_4$,
$R^8$ est un groupe alkyle en $C_1$ à $C_6$,
$R^9$ et $R^{10}$ représentent, indépendamment l'un de l'autre, de l'hydrogène ou un groupe alkyle en $C_1$ à $C_4$,
Hal est un halogène,
ainsi que leurs sels et leurs N-oxydes acceptables du point de vue physiologique.

**8.** Procédé de production de pyridylméthylcétones de formule V,

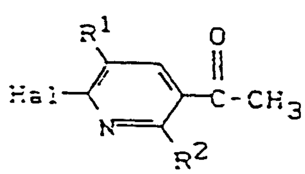

V

dans laquelle
$R^1$, $R^2$, Hal ont les définitions indiquées dans la revendication 7,
caractérisé en ce qu'on fait réagir des composes de formule VII,

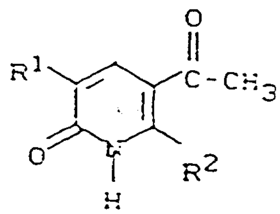

VII

dans laquelle
$R^1$ et $R^2$ ont la définition indiquée ci-dessus,
avec l'oxychlorure de phosphore, éventuellement en présence d'accepteurs d'acides.

**9.** Compositions pour stimuler la croissance d'animaux, caractérisées par une teneur en pyridyléthanolamines substituées de formule I suivant la revendication 1.

**10.** Utilisation de pyridyléthanolamines substituées de formule I suivant la revendication 1 pour stimuler la croissance d'animaux.

**11.** Composé de formule